# EUROPEAN PATENT APPLICATION

(11) **EP 3 456 565 A1**
(43) Date of publication of application: **20.03.2019**
(21) Application number: 18192406.9
(22) Date of filing: 04.09.2018
(51) Int. Cl.: B60H 3/00, B60N 2/56

(54) **AROMA SUPPLY DEVICE FOR A VEHICLE**

(30) Priority: 08.09.2017 JP 2017173334
(71) Applicant: TOYOTA JIDOSHA KABUSHIKI KAISHA, Toyota-shi, Aichi-ken, 471-8571 (JP)
(72) Inventor: GOTO, Takaaki, TOYOTA-SHI, AICHI-KEN, 471-8571 (JP)
(74) Representative: Cabinet Beau de Loménie

(57) **Abstract**

An aroma supply device (100) for a vehicle, comprising an air conditioning control unit (10) configured to control an air conditioning unit (12) so as to supply ventilated air, a temperature and an air volume of which are controlled, to specified regions of a vehicle cabin interior, an aroma generation unit (22) configured to aromatize the ventilated air, a seat ventilation unit (14) configured to suck air in through an air hole formed at a front face side of at least one of a seat cushion or a seat back and exhaust the air through an exhaust hole provided separately from the air hole, and an aroma control unit (20) configured to control the aroma generation unit (22) so as to alter a strength of aromatization in accordance with the regions to which the ventilated air is supplied and in accordance with operation of the seat ventilation unit (14).

## Description

### BACKGROUND

### Technical Field

Preferred embodiments relate to an aroma supply device for a vehicle.

### Related Art

A preferable aroma is diffused inside a vehicle to improve mood of vehicle occupants or to suppress (mask) the sensation of an undesirable smell inside a vehicle cabin.

Heretofore, an aroma has been dispersed inside a vehicle cabin using a commercially available air freshener. However, it takes time for a commercially available air freshener to thoroughly disperse an aroma in a vehicle cabin.

Japanese Patent Application Laid-Open (JP-A) No. 2004-268704 discloses the invention of an aroma generation device for a vehicle equipped with an air-blowing unit that generates a flow of air carrying an aroma generated by an aroma generator.

However, it is presumed that the aroma generation device for a vehicle disclosed in JP-A No. 2004-268704 is to be operated when required to help vehicle occupants relax, such as when the moods of vehicle occupants are bad, when an accident has occurred, or the like. The device is not controlled in conjunction with air conditioning of the vehicle.

### SUMMARY

In consideration of the circumstances described above, preferred embodiments provide an aroma supply device for a vehicle that can be controlled in conjunction with air conditioning of the vehicle.

An aroma supply device for a vehicle according to a first aspect of the present disclosure includes: an air conditioning control unit that controls an air conditioning unit so as to supply ventilated air, a temperature and an air volume of which are controlled, to specified regions of a vehicle cabin interior; an aroma generation unit that aromatizes the ventilated air; a seat ventilation unit that sucks air in through an air hole formed at a front face side of at least one of a seat cushion or a seat back and exhausts air through an exhaust hole provided separately from the air hole; and an aroma control unit that controls the aroma generation unit so as to alter a strength of aromatization in accordance with the regions to which the ventilated air is supplied and in accordance with operation of the seat ventilation unit.

The aroma supply device for a vehicle according to the first aspect may improve efficiency of emission of an aromatic component by altering the strength of aromatization of the ventilated air in accordance with the regions to which the ventilated air is supplied by the air conditioning and in accordance with operation of the seat ventilation unit.

In an aroma supply device for a vehicle according to a second aspect of the present disclosure, in the aroma supply device for a vehicle according to the first aspect, the aroma control unit controls the aroma generation unit so as to lower the strength of aromatization when a number of the regions is smaller and so as to raise the strength of aromatization when the number of the regions is larger.

The aroma supply device for a vehicle according to the second aspect may alter the strength of aromatization in accordance with the number of regions to which the ventilated air is supplied by the air conditioning.

In an aroma supply device for a vehicle according to a third aspect of the present disclosure, in the aroma supply device for a vehicle according to the first aspect or the second aspect, the aroma control unit controls the aroma generation unit so as to lower the strength of aromatization when the seat ventilation unit is operating and so as to raise the strength of aromatization when the seat ventilation unit is not operating.

The aroma supply device for a vehicle according to the third aspect may reduce the strength of the aromatization when the seat ventilation unit is operating.

In an aroma supply device for a vehicle according to a fourth aspect of the present disclosure, in the aroma supply device for a vehicle according to the third aspect, the seat ventilation unit is capable of altering an air volume of ventilation by the suction and exhaust, and the aroma control unit controls the aroma generation unit so as to lower the strength of aromatization when the air volume of the ventilation is larger and so as to raise the strength of aromatization when the air volume of the ventilation is smaller.

The aroma supply device for a vehicle according to the fourth aspect may alter the strength of the aromatization in accordance with the air volume of the ventilation by the seat ventilation unit.

According to the aroma supply device for a vehicle of the first aspect, because the strength of aromatization of the ventilated air is altered in accordance with the regions to which the ventilated air is supplied by the air conditioning and in accordance with operation of the seat ventilation unit, emission of the aromatic component may be made more efficient. Additionally, the aromatization may be controlled in conjunction with the air conditioning of the vehicle.

According to the aroma supply device for a vehicle of the second aspect, because the strength of aromatization is reduced when the number of regions to which the ventilated air is supplied by the air conditioning is small, emission of the aromatic component may be made more efficient and the aromatic component may be consumed efficiently.

According to the aroma supply device for a vehicle of the third aspect, because the strength of aromatization is reduced when the seat ventilation unit is operating, emission of the aromatic component may be made more efficient and the aromatic component may be consumed efficiently.

According to the aroma supply device for a vehicle of the fourth aspect, because the strength of aromatization is reduced when the air volume of the ventilation by the seat ventilation unit is large, emission of the aromatic component may be made more efficient and the aromatic component may be consumed efficiently.

### BRIEF DESCRIPTION OF THE DRAWINGS

Preferred embodiments will be described in detail based on the following figures, wherein:
Fig. 1 is a block diagram showing an example of structures of an aroma supply device for a vehicle according to a first exemplary embodiment of the present invention;
Fig. 2A is a schematic diagram showing an aroma generation device according to the first exemplary embodiment of the present invention, depicting an example of the aroma generation device that heats an aromatic component charged in a cartridge with a heating element and emits the aromatic component;
Fig. 2B is a schematic diagram showing the aroma generation device according to the first exemplary embodiment of the present invention, depicting an example of the aroma generation device that emits an aromatic component charged in a cartridge by ventilating air;
Fig. 3 is a schematic diagram showing an example of overall structure of a vehicle seat equipped with a seat ventilation system;
Fig. 4 is an example of a functional block diagram of the aroma supply device for a vehicle according to the first exemplary embodiment of the present invention; and
Fig. 5 is an example of a functional block diagram of an aroma supply device for a vehicle according to a second exemplary embodiment of the present invention.

### DETAILED DESCRIPTION

### - First Exemplary Embodiment -

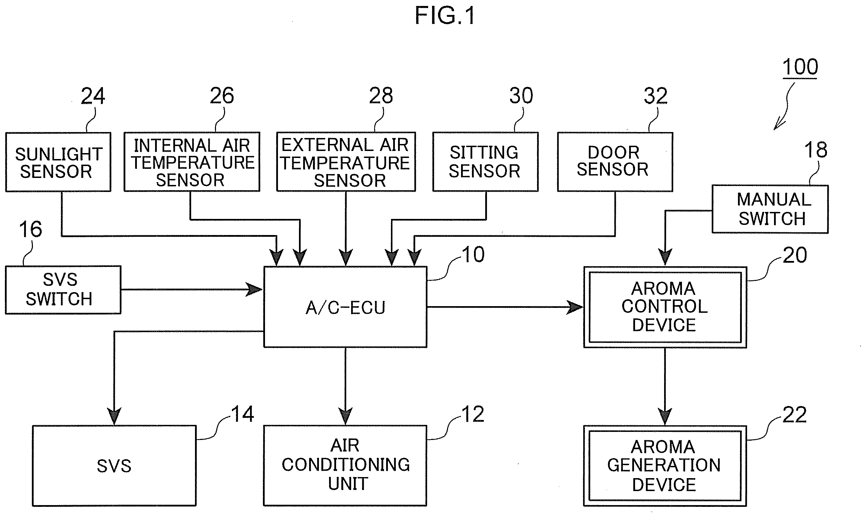

Herebelow, an aroma supply device for a vehicle 100 in accordance with the present exemplary embodiment is described using Fig. 1 to Fig. 4. Fig. 1 is a block diagram showing an example of structures of the aroma supply device for a vehicle 100 according to the present exemplary embodiment. The aroma supply device for a vehicle 100 shown in Fig. 1 operates in conjunction with air conditioning of the vehicle and includes an aroma generation device 22 and an aroma control device 20. The aroma generation device 22 emits an aromatic component to a vehicle cabin interior through an air vent. The aroma control device 20 controls the aroma generation device 22. The aroma control device 20 is connected to an air conditioning electronic control unit (ECU) 10 (below referred to as "the A/C ECU 10") and a manual switch 18. The A/C ECU 10 controls air conditioning of the vehicle. The manual switch 18, when operated by a user, turns the aroma generation device 22 on or off. The aroma control device 20 operates in conjunction with the air conditioning of the vehicle in accordance with control signals from the A/C ECU 10.

The A/C ECU 10 is a control device that controls an air conditioning unit 12 and a seat ventilation system (SVS) 14. The SVS 14 sucks air from inside the vehicle cabin through a sitting surface and a rear surface of a seat and exhausts the air to below and rearward of the seat. The A/C ECU 10 is connected to an SVS switch 16 that turns the SVS 14 on or off, a sunlight sensor 24 that measures the strength of sunlight, an internal air temperature sensor 26 that detects an air temperature of the vehicle cabin interior, an external air temperature sensor 28 that detects an external air temperature, a sitting sensor 30 that detects an occupant sitting on the seat, and a door sensor 32 that detects opening and closing of a door of the vehicle. The A/C ECU 10 controls the air conditioning unit 12 and the SVS 14 in accordance with signals inputted from this group of switches and sensors. The air conditioning unit 12 according to the present exemplary embodiment is capable of concentrating airflows at specific regions, such as a driver seat, front seats that are the driver seat and a front passenger seat, and the like.

The A/C ECU 10 outputs information relating to control of the air conditioning unit 12 and SVS 14 to the aroma control device 20.

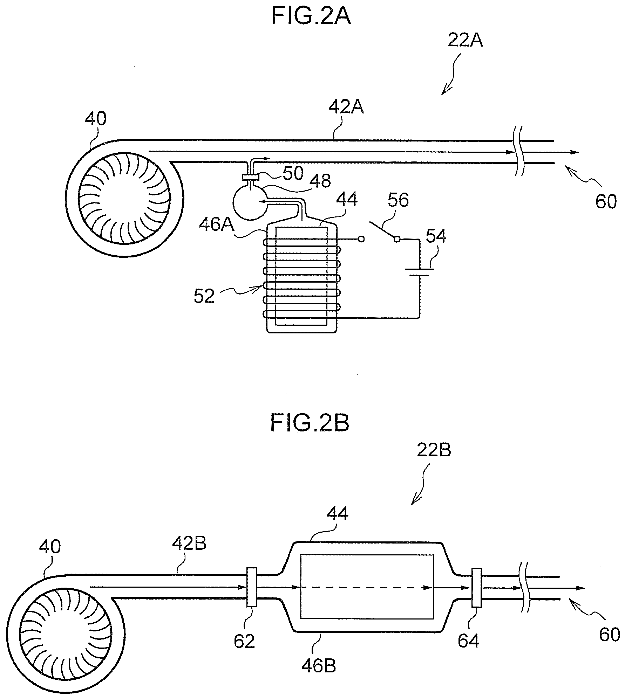

Fig. 2A and Fig. 2B are schematic diagrams showing the aroma generation device 22 according to the present exemplary embodiment. Fig. 2A depicts an example that is an aroma generation device 22A, which heats an aromatic component charged in a cartridge 44 with a heating element 52 and emits the aromatic component. Fig. 2B depicts an example that is an aroma generation device 22B, which emits the aromatic component charged in the cartridge 44 by ventilating air.

In the aroma generation device 22A shown in Fig. 2A, the heating element 52 is heated by electric power from a battery 54 that is turned on by a switch 56, and heats a canister 46A in which the cartridge 44 is loaded. In the cartridge 44, for example, a fragrance is charged into a porous, chemically inactive carrier that is suitable for adsorption of the fragrance. For example, any of a silica gel, alumina, diatomaceous earth or the like can be used as a carrier.

The charged aromatic component is volatilized from the heated cartridge 44 and the volatilized aromatic component is reserved in a reservoir 48. Gas containing the aromatic component inside the canister 46A and the reservoir 48 is kept at a positive pressure relative to atmospheric pressure by the heating of the heating element 52.

A ventilator 40 structured with a multi-vane fan such as a sirocco fan or the like is turned by an electric motor. If an electromagnetic valve 50 is open in a state in which air is flown in a ventilation channel 42A by the ventilator 40, the aromatic component is jetted out from the reservoir 48, which is in the positive pressure state due to the heating, to the ventilation channel 42A. Although not shown in the drawings, air in the ventilation channel 42A that is aromatized with the aromatic component from the cartridge 44 is mixed with air-conditioned air ventilated into the vehicle cabin by the air conditioning unit 12 and is ultimately emitted to the vehicle cabin interior through an air vent 60 of the air conditioning unit 12.

Although many aromatic components of fragrances are liquid or solid at room temperature, they may be gasified or sublimated by heating and dispersed in air. Thus, ventilated air from the air conditioning unit 12 may be aromatized and emitted into the vehicle cabin by the aroma generation device 22A shown in Fig. 2A. Moreover, the aroma generation device 22A may alter an aroma blowing strength, which is a strength of aromatization of the ventilated air from the air vent 60, by altering an opening angle of the electromagnetic valve 50. When the opening angle of the electromagnetic valve 50 is larger, the aroma blowing strength is larger, and when the opening angle of the electromagnetic valve 50 is smaller, the aroma blowing strength is smaller.

However, if a component that is vulnerable to heat is present in a fragrance, electrical heating is not appropriate. In this case, the aroma generation device 22B shown in Fig. 2B is employed. The aroma generation device 22B shown in Fig. 2B passes ventilated air from the ventilator 40 to the cartridge 44 inside a canister 46B, which is provided in a ventilation channel 42B, and the charged aromatic component is emitted. An electromagnetic valve 62 is provided at one side of the canister 46B at which the ventilator 40 is disposed, and an electromagnetic valve 64 is provided at another side of the canister 46B at which the air vent 60 is disposed. When the aromatic component is not to be emitted into the vehicle cabin, both the electromagnetic valves 62 and 64 are closed. Consequently, wasteful emission of the aromatic component charged into the cartridge 44 is prevented and only ventilated air from the air conditioning unit 12 is emitted into the vehicle cabin through the air vent 60.

In the aroma generation device 22B shown in Fig. 2B, the cartridge 44 is not heated and there is no risk of an aromatic component that is vulnerable to heat to decompose. Therefore, a greater range of fragrances may be used than in the aroma generation device 22A shown in Fig. 2A. Moreover, by altering opening angles of the electromagnetic valves 62 and 64, the aroma generation device 22B may alter the aroma blowing strength, which is the strength of aromatization of the ventilated air from the air vent 60. When the opening angles of the electromagnetic valves 62 and 64 are larger, the aroma blowing strength is larger, and when the opening angles of the electromagnetic valves 62 and 64 are smaller, the aroma blowing strength is smaller. Further still, the aroma blowing strength may be altered by altering a rotating speed of the fan of the ventilator 40. When the rotating speed of the fan of the ventilator 40 is greater, air amounts passing through the cartridge 44 are increased and consequently the aroma blowing strength is larger. When the rotating speed of the fan of the ventilator 40 is smaller, air amounts passing through the cartridge 44 are reduced and consequently the aroma blowing strength is smaller.

In the aroma generation devices 22A and 22B shown in Fig. 2A and Fig. 2B, the single cartridge 44 is employed; that is, one type of fragrance is employed. However, plural cartridges charged with respectively different fragrances may be included, and any one of the plural cartridges may be selected and employed.

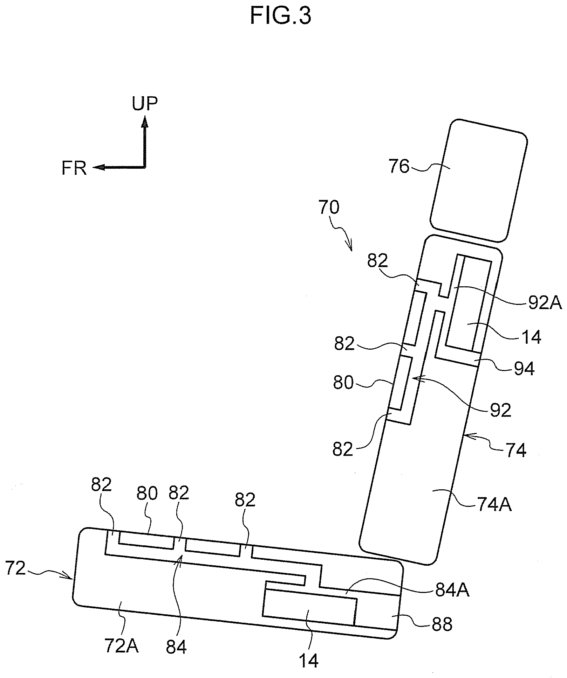

Fig. 3 is a schematic diagram showing an example of overall structure of a vehicle seat 70 equipped with the SVS 14. As shown in Fig. 3, the vehicle seat 70 is provided with a seat cushion 72 and a seat back 74. The seat cushion 72 supports a buttock area and thigh area of a seated occupant. The seat back 74 is provided so as to extend upward from a rear end portion of the seat cushion 72 and supports a waist area and back area of the occupant. A headrest 76 that supports a head area of the occupant is provided at an upper end portion of the seat back 74. The seat cushion 72 and the seat back 74 are an example of a seat main body.

The seat cushion 72 and seat back 74 are each provided with a surface skin 80 that covers a front face (a face on which the occupant sits) and that features air permeability.

The seat cushion 72 is provided with a main body portion 72A. Although not shown in the drawings, the main body portion 72A includes a seat frame, and a seat pad that is supported by the seat frame. The seat cushion 72 is further provided with plural air holes 82 formed in the front face of the main body portion 72A and an airflow channel 84 in an interior of the main body portion 72A. The airflow channel 84 is in fluid communication with each of the plural air holes 82. Air entering through the air holes 82 flows through the airflow channel 84.

The airflow channel 84 is a structure that brings air entering through the plural air holes 82 together inside the main body portion 72A. The airflow channel 84 is provided with an accommodation portion 84A at which the channel is enlarged, at a downstream side in a flow direction in which the air flows together. The SVS 14 is provided in the accommodation portion 84A to serve as a ventilator that sucks air in through the air holes 82 into the airflow channel 84. That is, the SVS 14 is disposed in the interior of the main body portion 72A.

An exhaust vent 88 is provided at a rear face side of the main body portion 72A. The exhaust vent 88 is in fluid communication with the accommodation portion 84A and exhausts air blown out from the SVS 14 to outside the main body portion 72A. The exhaust vent 88 is covered by the surface skin 80, which is disposed at a rear face of the main body portion 72A and features air permeability. When the SVS 14 operates, air is sucked in through the plural air holes 82 via the surface skin 80 to the airflow channel 84, and then air blown out from the SVS 14 is exhausted to the exterior through the exhaust vent 88 via the surface skin 80.

The seat back 74 has a substantially similar structure to the seat cushion 72. A detailed description of the seat back 74 is not given here, but the seat back 74 is provided with a main body portion 74A, plural air holes 82 formed in the front face of the main body portion 74A, and an airflow channel 92 inside the main body portion 74A through which air entering through the plural air holes 82 flows. The seat back 74 is further provided with another of the SVS 14, and an exhaust vent 94 at a rear face side of the main body portion 74A. The SVS 14 is disposed in the main body portion 74A in an accommodation portion 92A at a downstream side in the flow direction of the airflow channel 92. Air blown out from the SVS 14 is exhausted through the exhaust vent 94. The exhaust vent 94 is covered by the surface skin 80, which is disposed at the rear face of the main body portion 74A.

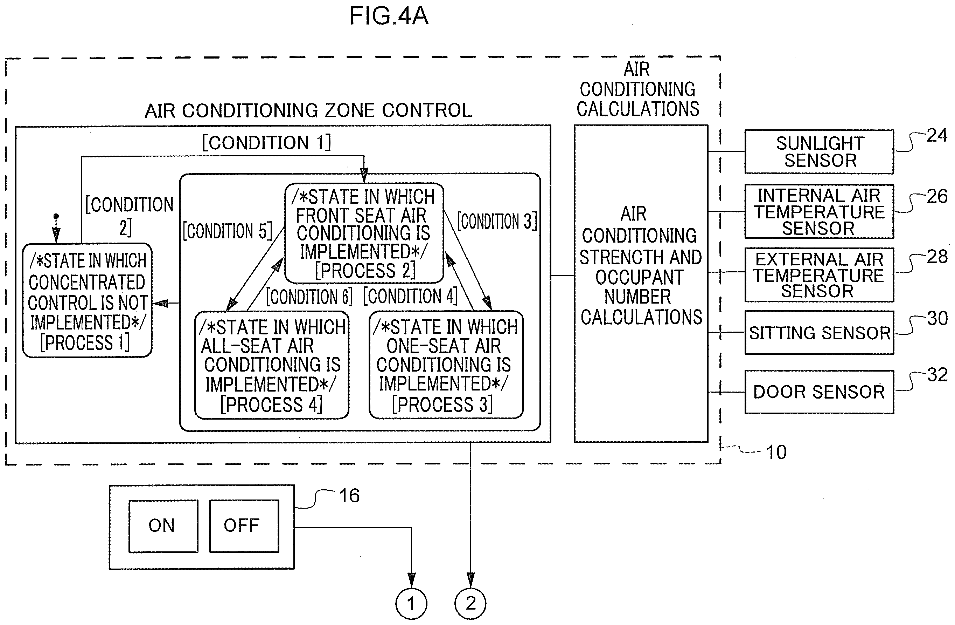

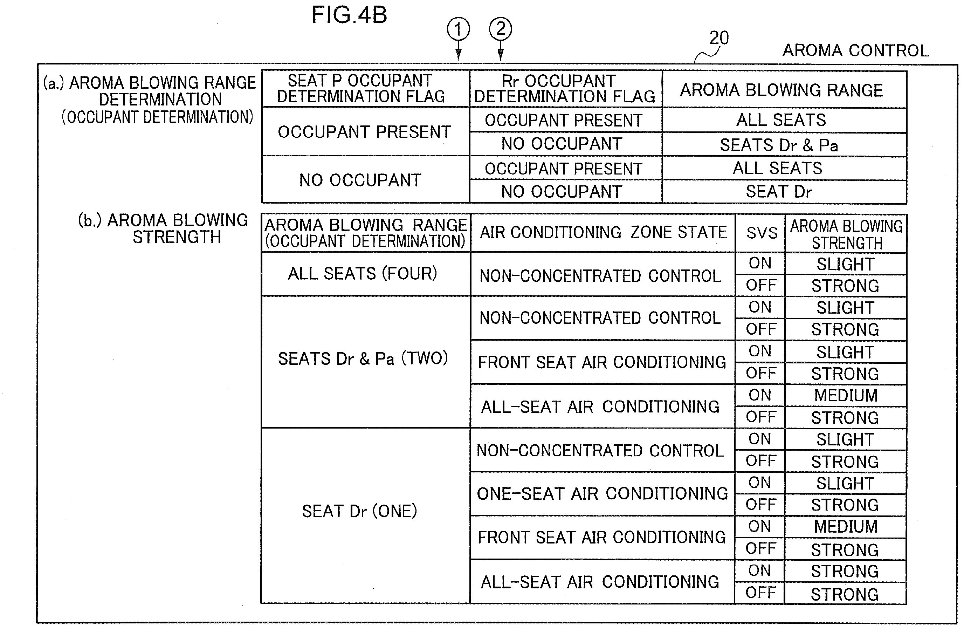

Fig. 4 is an example of a functional block diagram of the aroma supply device for a vehicle 100 according to the present exemplary embodiment. In the present exemplary embodiment, as described above, signals are inputted to the A/C ECU 10 from the sunlight sensor 24, the internal air temperature sensor 26, the external air temperature sensor 28, each sitting sensor 30 and the door sensor 32. The A/C ECU 10 calculates an air conditioning strength, which is an operational degree of the air conditioning unit 12, on the basis of the signals inputted from the sunlight sensor 24, the internal air temperature sensor 26, the external air temperature sensor 28 and the door sensor 32. The A/C ECU 10 also performs an occupant number calculation to calculate a number of vehicle occupants on the basis of the signals from the sitting sensors 30.

For the air conditioning strength, for example, a cooling degree is greater when sunlight detected by the sunlight sensor 24 is stronger, and the cooling degree is greater when respective temperatures dictated by the internal air temperature sensor 26 and the external air temperature sensor 28 are higher. When the door of the vehicle is open according to the door sensor 32, a cooling or heating degree is raised to prevent the temperature inside the vehicle departing from a comfortable range. On the basis of the results of the air conditioning strength and occupant number calculations, the A/C ECU 10 performs air conditioning calculations to control the temperature and air volume of ventilated air from the air conditioning unit 12.

Subsequent to the air conditioning calculations, the A/C ECU 10 executes air conditioning zone control. The air conditioning zone control is broadly divided into concentrated control (processes 2, 3 and 4) to implement concentrated blowing of air at specified regions (occupants sitting regions), and non-concentrated control (process 1) that does not implement concentrated control but circulates ventilated air through the whole of the vehicle cabin interior. The mode of the concentrated control can further be altered in accordance with application of conditions 2, 3, 4, 5 and 6 after the non-concentrated control is transitioned to the concentrated control (which transition is condition 1) in accordance with the operation of switches relating to air conditioning on an instrument panel.

As shown in Fig. 4, after switching from the non-concentrated control to the concentrated control in accordance with condition 1, front seat air conditioning (process 2) to concentrate the air conditioning on front seats (the driver seat and the front passenger seat) is implemented. From this front seat air conditioning state, control may be switched to one-seat air conditioning (process 3) in accordance with condition 3, to concentrate ventilated air on the driver seat, or to all-seat air conditioning (process 4) in accordance with condition 5, to concentrate ventilated air on all seats including rear seats.

From the one-seat air conditioning, control may be switched to the front seat air conditioning to concentrate the air conditioning on the driver seat and the front passenger seat in accordance with condition 4 for concentrating ventilated air on the front seats. From the all-seat air conditioning, control may be switched to the front seat air conditioning in accordance with condition 6 for concentrating ventilated air on the front seats.

The air conditioning unit 12 relating to the present exemplary embodiment includes separate air vents for the driver seat, the front passenger seat and the rear seats. The A/C ECU 10 implements the one-seat air conditioning, the front seat air conditioning or the all-seat air conditioning by controlling to select the air vents.

Control may also be switched from the concentrated control state to the non-concentrated control in accordance with condition 2 for cancelling the concentrated control. As described below, in the present exemplary embodiment, the strength of aromatization is altered in accordance with a number of regions (seats) at which concentrated control is directed. Thus, as an example of information relating to air-conditioning zone control, a number of regions at which concentrated control is directed is defined as "one" for one-seat air conditioning, "two" for front seat air conditioning, and "three" for all-seat air conditioning. In the case of non-concentrated control, the number of regions at which concentrated control is directed is defined as the minimum value, "zero".

Signals relating to whether the SVS 14 is on or off are inputted to the A/C ECU 10 from the SVS switch 16. The A/C ECU 10 inputs the above-mentioned information relating to air conditioning zone control and signals relating to operation of the SVS switch 16 to the aroma control device 20.

The aroma control device 20 implements (a.) an aroma blowing range determination (occupant determination) on the basis of signals relating to detection results from the sitting sensors 30 that are inputted from the A/C ECU 10 to the aroma control device 20. For this occupant determination, firstly, a seat P occupant determination flag is set in relation to whether or not there is an occupant on the front passenger seat (seat P). The seat P occupant determination flag is set to "occupant present" when an occupant is on the front passenger seat and is set to "no occupant" when there is no occupant on the front passenger seat.

Further for the occupant determination, an Rr occupant determination flag is set in relation to whether or not there is an occupant on a rear seat (Rr). The Rr occupant determination flag is set to "occupant present" when an occupant is on the rear seat and is set to "no occupant" when there is no occupant on the rear seat.

The aroma blowing range is specified on the basis of the above-described seat P occupant determination flag and Rr occupant determination flag. The aroma blowing range is specified as follows: to all seats if an occupant is present on the front passenger seat and an occupant is present on the rear seat; to the driver seat (Dr) and the front passenger seat if an occupant is present on the front passenger seat but no occupant is on the rear seat; to all seats if no occupant is on the front passenger seat but an occupant is present on the rear seat; and to the driver seat if no occupant is on the front passenger seat and no occupant is on the rear seat.

Then, the aroma control device 20 specifies (b.) an aroma blowing strength in accordance with the above-described aroma blowing range (a.), the air conditioning zone control state, and the operation state of the SVS 14. For example, the aroma blowing strength is specified as follows.

If the aroma blowing range is all seats, the air conditioning zone control state is non-concentrated control, and the SVS 14 is on, then the aroma blowing strength is set to "slight". If the aroma blowing range is all seats, the air conditioning zone control state is non-concentrated control, and the SVS 14 is off, then the aroma blowing strength is set to "strong".

If the aroma blowing range is the driver seat and front passenger seat, the air conditioning zone control state is non-concentrated control, and the SVS 14 is on, then the aroma blowing strength is set to "slight". If the aroma blowing range is the driver seat and front passenger seat, the air conditioning zone control state is non-concentrated control, and the SVS 14 is off, then the aroma blowing strength is set to "strong".

If the aroma blowing range is the driver seat and front passenger seat, the air conditioning zone control state is front seat air conditioning, and the SVS 14 is on, then the aroma blowing strength is set to "slight". If the aroma blowing range is the driver seat and front passenger seat, the air conditioning zone control state is front seat air conditioning, and the SVS 14 is off, then the aroma blowing strength is set to "strong".

If the aroma blowing range is the driver seat and front passenger seat, the air conditioning zone control state is all-seat air conditioning, and the SVS 14 is on, then the aroma blowing strength is set to "medium". If the aroma blowing range is the driver seat and front passenger seat, the air conditioning zone control state is all-seat air conditioning, and the SVS 14 is off, then the aroma blowing strength is set to "strong".

If the aroma blowing range is the driver seat, the air conditioning zone control state is non-concentrated control, and the SVS 14 is on, then the aroma blowing strength is set to "slight". If the aroma blowing range is the driver seat, the air conditioning zone control state is non-concentrated control, and the SVS 14 is off, then the aroma blowing strength is set to "strong".

If the aroma blowing range is the driver seat, the air conditioning zone control state is one-seat air conditioning, and the SVS 14 is on, then the aroma blowing strength is set to "slight". If the aroma blowing range is the driver seat, the air conditioning zone control state is one-seat air conditioning, and the SVS 14 is off, then the aroma blowing strength is set to "strong".

If the aroma blowing range is the driver seat, the air conditioning zone control state is front seat air conditioning, and the SVS 14 is on, then the aroma blowing strength is set to "medium". If the aroma blowing range is the driver sea, the air conditioning zone control state is front seat air conditioning, and the SVS 14 is off, then the aroma blowing strength is set to "strong".

If the aroma blowing range is the driver seat, the air conditioning zone control state is all-seat air conditioning, and the SVS 14 is on, then the aroma blowing strength is set to "strong". If the aroma blowing range is the driver seat, the air conditioning zone control state is all-seat air conditioning, and the SVS 14 is off, then the aroma blowing strength is set to "strong".

In the present exemplary embodiment, when the number of seats at which concentrated control of the air conditioning zone is directed is small, the aroma blowing strength is lowered, and when the number of seats at which concentrated control is directed is large, the aroma blowing strength is raised. When the number of seats at which concentrated control is directed is large, the aromatization strength at the aroma generation device 22 needs to be raised because the aromatized ventilated air is being distributed to the large number of seats. When the number of seats at which concentrated control is directed is small, because the aromatized ventilated air is supplied toward a limited number of seats, enough of the aromatic component will reach the designated seat occupants even if the aromatization strength at the aroma generation device 22 is lowered.

In the present exemplary embodiment, when the SVS 14 is on, the aroma blowing strength is set lower than when the SVS 14 is off. This is because airflow from a front side to behind of an occupant is produced when the SVS 14 is on. Therefore, the aromatic component supplied from the aroma generation device 22 is more likely to reach an occupant than when the SVS 14 is off. In this case, the occupant may sense the aroma sufficiently even when the aroma blowing strength is lowered.

In the present exemplary embodiment, if the air conditioning zone control state can be altered as appropriate by switch operation by an occupant, then the aroma blowing range (a.) determined on the basis of detection results from the sitting sensors 30 may not match the air conditioning zone control state. In this case, the present exemplary embodiment calculates the aroma blowing strength as illustrated in Fig. 4 on the basis of a first principle that the aroma blowing strength is lower when the number of seats at which concentrated control is directed is small and the aroma blowing strength is higher when the number of seats at which concentrated control is directed is large, and a second principle that the aroma blowing strength is lower when the SVS 14 is on than when the SVS 14 is off. Thus, the aromatic component charged in the cartridge 44 is emitted efficiently.

As described above, the aroma supply device for a vehicle 100 according to the present exemplary embodiment may aromatize ventilated air and excellently supply the aromatic component to seats at which concentrated control of the air conditioning is directed, in conjunction with the air conditioning. The aroma supply device for a vehicle 100 may suppress consumption of the fragrance charged in the cartridge 44 by lowering the aroma blowing strength when the number of seats at which concentrated control is directed is small.

The aroma supply device for a vehicle 100 according to the present exemplary embodiment may also suppress consumption of the fragrance charged in the cartridge 44 by lowering the aroma blowing strength when the SVS 14 is on, because enough of the aromatic component can reach occupants even though the aroma blowing strength is lowered.

### - Second Exemplary Embodiment -

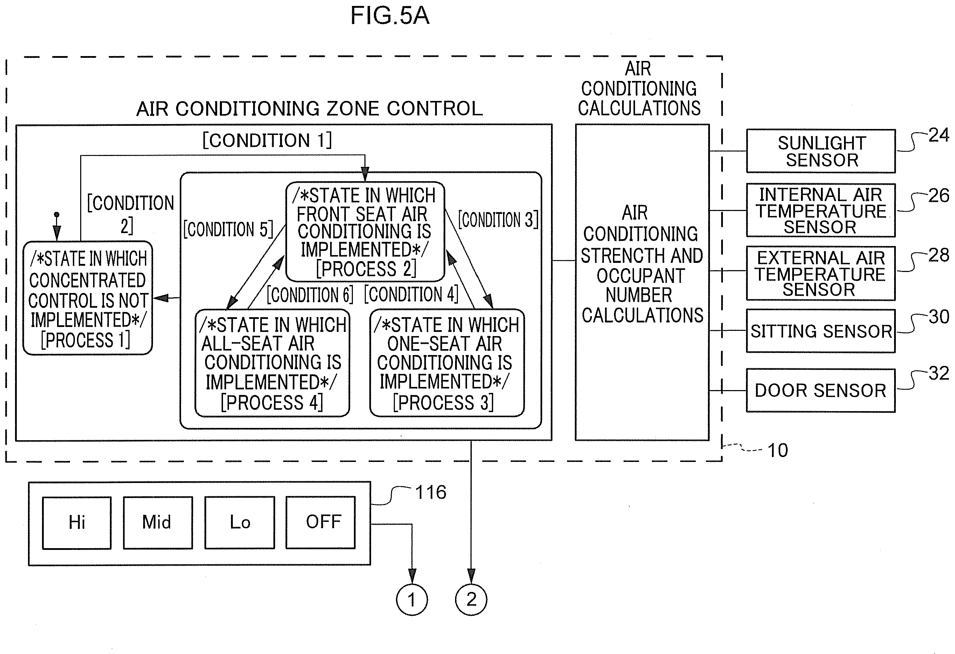

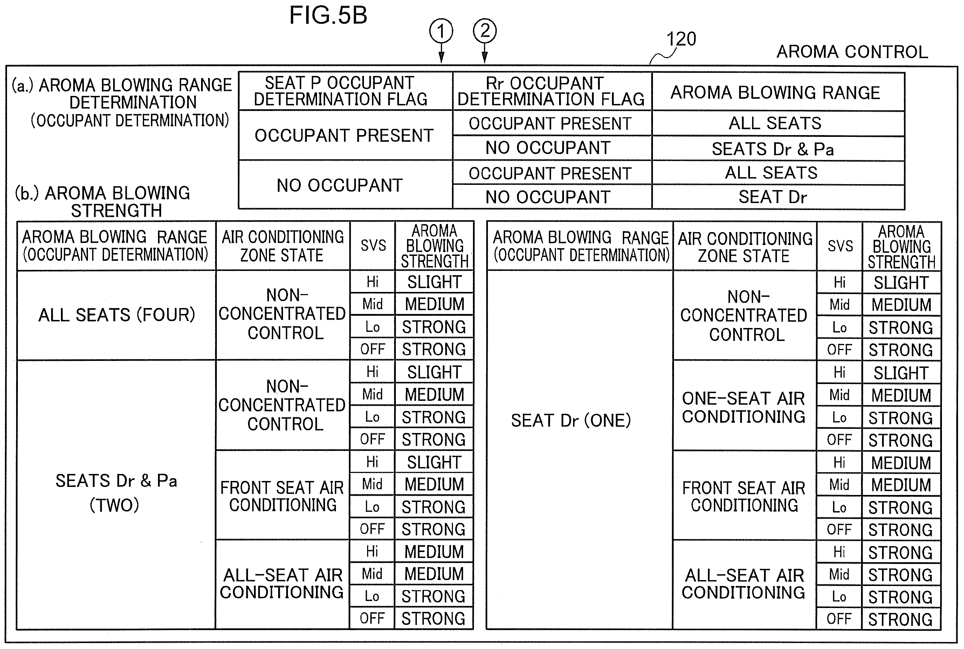

Now, a second exemplary embodiment of the present invention is described using Fig. 5. The present exemplary embodiment differs from the first exemplary embodiment in regard to the configuration of an SVS switch 116 and (b.) the aroma blowing out strength of an aroma control device 120, but is similar to the first exemplary embodiment in other structures and controls. Accordingly, the same reference symbols as in the first exemplary embodiment are applied to structures that are the same as in the first exemplary embodiment, and detailed descriptions thereof are not given.

Fig. 5 is an example of a functional block diagram of an aroma supply device for a vehicle according to the present exemplary embodiment. Unlike the SVS switch 16 according to the first exemplary embodiment that has two selections, on and off, the SVS switch 116 is configured to be switchable between the respective positions "OFF" to turn the SVS 14 off, "Lo" to set ventilation by the SVS 14 to a low strength, "Mid" to set the ventilation to a medium strength, and "Hi" to set the ventilation to a high strength. Accordingly, in accordance with positions of the SVS switch 116, (b.) the aroma blowing strength in the present exemplary embodiment can be set in more numerous modes than in the first exemplary embodiment.

Specification of the aroma blowing strength is described below. Control up to the control of (a.) the aroma blowing range determination (occupant determination) is the same as in the first exemplary embodiment, so is not described in detail.

If the aroma blowing range is all seats and the air conditioning zone control state is non-concentrated control, correspondences of positions of the SVS switch 116 and aroma blowing strengths are as follows. If the SVS switch 116 is set to "Hi", the aroma blowing strength is set to "slight", if the SVS switch 116 is set to "Mid", the aroma blowing strength is set to "medium", and if the SVS switch 116 is set to "Lo" or "OFF", the aroma blowing strength is set to "strong". When the strength of ventilation by the SVS 14 is high, the total amount of the aromatic component reaching an occupant is large, so the aroma blowing strength is lowered in this case. When the strength of ventilation by the SVS 14 is low, the total amount of the aromatic component reaching an occupant is small, so the aroma blowing strength is raised in this case.

If the aroma blowing range is the driver seat and front passenger seat and the air conditioning zone control state is non-concentrated control, correspondences of positions of the SVS switch 116 and aroma blowing strengths are as follows. If the SVS switch 116 is set to "Hi", the aroma blowing strength is set to "slight", if the SVS switch 116 is set to "Mid", the aroma blowing strength is set to "medium", and if the SVS switch 116 is set to "Lo" or "OFF", the aroma blowing strength is set to "strong".

If the aroma blowing range is the driver seat and front passenger seat and the air conditioning zone control state is front seat air conditioning, correspondences of positions of the SVS switch 116 and aroma blowing strengths are as follows. If the SVS switch 116 is set to "Hi", the aroma blowing strength is set to "slight", if the SVS switch 116 is set to "Mid", the aroma blowing strength is set to "medium", and if the SVS switch 116 is set to "Lo" or "OFF", the aroma blowing strength is set to "strong".

If the aroma blowing range is the driver seat and front passenger seat and the air conditioning zone control state is all-seat air conditioning, correspondences of positions of the SVS switch 116 and aroma blowing strengths are as follows. If the SVS switch 116 is set to "Hi" or "Mid", the aroma blowing strength is set to "medium", and if the SVS switch 116 is set to "Lo" or "OFF", the aroma blowing strength is set to "strong".

This is because in the present exemplary embodiment, similarly to the first exemplary embodiment, when the number of seats at which concentrated control is directed is small, the aroma blowing strength is lowered, and when the number of seats at which concentrated control is directed is large, the aroma blowing strength is raised. As a result, the aromatic component is emitted into the vehicle cabin efficiently.

If the aroma blowing range is the driver seat and the air conditioning zone control state is non-concentrated control or one-seat air conditioning, correspondences of positions of the SVS switch 116 and aroma blowing strengths are as follows. If the SVS switch 116 is set to "Hi", the aroma blowing strength is set to "slight", if the SVS switch 116 is set to "Mid", the aroma blowing strength is set to "medium", and if the SVS switch 116 is set to "Lo" or "OFF", the aroma blowing strength is set to "strong".

If the aroma blowing range is the driver seat and the air conditioning zone control state is front seat air conditioning, correspondences of positions of the SVS switch 116 and aroma blowing strengths are as follows. If the SVS switch 116 is set to "Hi" or "Mid", the aroma blowing strength is set to "medium", and if the SVS switch 116 is set to "Lo" or "OFF", the aroma blowing strength is set to "strong".

If the aroma blowing range is the driver seat and the air conditioning zone control state is all-seat air conditioning, the aroma blowing strength is set to "strong" regardless of the position of the SVS switch 116.

As described above, the aroma supply device for a vehicle according to the present exemplary embodiment may control aromatization in conjunction with the air conditioning in a greater number of modes than the first exemplary embodiment, in accordance with strengths of ventilation by the SVS 14.

In the structures of the attached claims, the air conditioning control unit corresponds to the A/C ECU 10, the aroma generation unit corresponds to the aroma generation device 22, the seat ventilation unit corresponds to the SVS 14, and the aroma control unit corresponds to the aroma control device 20.

## Claims

1. An aroma supply device (100) for a vehicle, comprising:
an air conditioning control unit (10) configured to control an air conditioning unit (12) so as to supply ventilated air, a temperature and an air volume of which are controlled, to specified regions of a vehicle cabin interior;
an aroma generation unit (22) configured to aromatize the ventilated air;
a seat ventilation unit (14) configured to suck air in through an air hole (82) formed at a front face side of at least one of a seat cushion (72) or a seat back (74) and exhaust the air through an exhaust hole (88, 94) provided separately from the air hole (82); and
an aroma control unit (20) configured to control the aroma generation unit (22) so as to alter a strength of aromatization in accordance with the regions to which the ventilated air is supplied and in accordance with operation of the seat ventilation unit (14).

2. The aroma supply device (100) for a vehicle according to claim 1, wherein the aroma control unit (20) is configured to control the aroma generation unit (22) so as to lower the strength of aromatization in a case in which a number of the regions is smaller and so as to raise the strength of aromatization in a case in which the number of the regions is larger.

3. The aroma supply device (100) for a vehicle according to claim 1 or claim 2, wherein the aroma control unit (20) is configured to control the aroma generation unit (22) so as to lower the strength of aromatization in a case in which the seat ventilation unit (14) is operating and so as to raise the strength of aromatization when the seat ventilation unit (14) is not operating.

4. The aroma supply device (100) for a vehicle according to claim 3, wherein:
the seat ventilation unit (14) is capable of altering an air volume of ventilation by the suction and exhaust; and
the aroma control unit (20) is configured to control the aroma generation unit (22) so as to lower the strength of aromatization when the air volume of the ventilation is larger and so as to raise the strength of aromatization when the air volume of the ventilation is smaller.
